# EUROPÄISCHE PATENTSCHRIFT

(11) **EP 1 794 238 B1**
(45) Veröffentlichungstag und Bekanntmachung des Hinweises auf die Patenterteilung: **30.07.2008**
(21) Anmeldenummer: 05792093.6
(22) Anmeldetag: 15.09.2005
(51) Int. Cl.: C09B 61/00

(54) **VERFAHREN ZUR HERSTELLUNG VON TROCKENPULVERN EINES ODER MEHRERER CAROTINOIDE**
METHOD FOR PRODUCING DRY POWDERS OF AT LEAST ONE CAROTENOID
PROCEDE DE PRODUCTION DE POUDRES SECHES D'UN OU DE PLUSIEURS CAROTENOIDES

(30) Priorität: 21.09.2004 DE 102004046026
(43) Veröffentlichungstag der Anmeldung: 13.06.2007
(73) Patentinhaber: BASF SE, 67056 Ludwigshafen (DE)
(72) Erfinder: MUSAEUS, Nina, DK-2900 Hellerup (DK); JENSEN, Carsten, Ninn, DK-2750 Ballerup (DK)
(86) Internationale Anmeldenummer: PCT/EP2005/009908
(87) Internationale Veröffentlichungsnummer: WO 2006/032399

(56) Entgegenhaltungen:
- EP-A- 0 864 326
- WO-A-91/06292
- DE-B- 1 211 911

## Beschreibung

Die Erfindung betrifft ein Verfahren zur Herstellung von Trockenpulvern eines oder mehrerer Carotinoide, insbesondere von Carotinoiden, ausgewählt aus der Gruppe, bestehend aus β-Carotin, Lutein, Zeaxanthin und Lycopin oder Mischungen davon.

Die Stoffklasse der Carotinoide klassifiziert man in zwei Hauptgruppen, die Carotine und die Xanthophylle. Im Unterschied zu den Carotinen, bei denen es sich um reine Polyen-Kohlenwasserstoffe handelt, wie beispielsweise β-Carotin oder Lycopin, kommen in den Xanthophyllen noch Sauerstoff-Funktionen wie Hydroxy-, Epoxy- und/oder Carbonylgruppen vor. Typische Vertreter dieser Gruppe sind u.a. Astaxanthin, Canthaxanthin, Lutein und Zeaxanthin.

Zu den Sauerstoff-haltigen Carotinoiden zählen auch Citranaxanthin und β-Apo-8'-Carotinsäureethylester.

Sauerstoff-haltige Carotinoide sind in der Natur weit verbreitet und kommen u.a. im Mais (Zeaxanthin), in grünen Bohnen (Lutein), in Paprika (Capsanthin), in Eidottern (Lutein) sowie in Krebsen und Lachsen (Astaxanthin) vor, wobei sie diesen Nahrungsmitteln ihre charakteristische Färbung verleihen.

Diese sowohl synthetisch zugänglichen als auch aus natürlichen Quellen isolierbaren Polyene stellen für die Lebens- und Futtermittelindustrie sowie für den pharmazeutischen Bereich wichtige Farb- und Wirkstoffe dar und sind, wie im Falle von Astaxanthin, Wirkstoffe mit Provitamin-A Aktivität beim Lachs.

Sowohl Carotine als auch Xanthophylle sind in Wasser unlöslich, während in Fetten und Ölen eine jedoch nur geringe Löslichkeit gefunden wird. Diese begrenzte Löslichkeit sowie die hohe Oxidationsempfindlichkeit stehen einer direkten Anwendung der durch chemische Synthese erhaltenen, relativ grobkörnigen Produkte in der Einfärbung von Lebens- und Futtermitteln entgegen, da die Substanzen in grobkristalliner Form nicht lagerstabil und nur schlechte Färbungsergebnisse liefern. Diese für die praktische Verwendung der Carotinoide nachteiligen Effekte wirken sich insbesondere im wässrigen Medium aus.

Nur durch gezielt hergestellte Formulierungen, in denen die Wirkstoffe in fein verteilter Form und gegebenenfalls durch Schutzkolloide oxidationsgeschützt vorliegen, lassen sich bei der direkten Einfärbung von Lebensmitteln verbesserte Farbausbeuten erzielen. Außerdem führen diese in Futtermitteln verwendeten Formulierungen zu einer höheren Bioverfügbarkeit der Carotinoide bzw. Xanthophylle und damit indirekt zu besseren Färbungseffekten z.B. bei der Eidotter- oder Fischpigmentierung.

Zur Verbesserung der Farbausbeuten und zur Erhöhung der Resorbierbarkeit bzw. Bioverfügbarkeit sind verschiedene Verfahren beschrieben worden, die alle das Ziel haben, die Kristallitgröße der Wirkstoffe zu verkleinern und auf einen Teilchengrößenbereich von kleiner 10 µm zu bringen.

Zahlreiche Methoden, u.a. beschrieben in Chimia 21, 329 (1967), WO 91/06292 sowie in WO 94/19411, bedienen sich dabei der Vermahlung von Carotinoiden mittels einer Kolloidmühle und erzielen damit Partikelgrößen von 2 bis 10 µm.

Daneben existieren eine Reihe von kombinierten Emulgier-/Sprühtrocknungsverfahren, wie sie z.B. in DE-A-12 11 911 oder in EP-A-0 410 236 beschrieben sind.

Gemäß der europäischen Patentschrift EP-B-0 065 193 erfolgt die Herstellung von feinverteilten, pulverförmigen Carotinoidpräparaten dadurch, dass man ein Carotinoid in einem flüchtigen, mit Wasser mischbaren organischen Lösungsmittel bei erhöhten Temperaturen, gegebenenfalls unter erhöhtem Druck löst, das Carotinoid durch Mischen mit einer wässrigen Lösung eines Schutzkolloids ausfällt und anschließend sprühtrocknet.

Ein analoges Verfahren zur Herstellung von feinverteilten, pulverförmigen Carotinoidpräparaten wird in EP-A-0 937 412 unter Verwendung von mit Wasser nicht mischbaren Lösungsmitteln beschrieben.

DE-A-44 24 085 beschreibt die Verwendung von teilabgebauten Sojaproteinen als Schutzkolloide für fettlösliche Wirkstoffe. Die hier offenbarten Sojaproteine weisen einen Abbaugrad von 0,1 bis 5 % auf.

In der deutschen Offenlegungsschrift DE-A-101 04 494 wird die Herstellung von Carotinoid Trockenpulvern unter Verwendung von Sojaproteinen zusammen mit Lactose als Schutzkolloide beschrieben.

Trotz der im eingangs genannten Stand der Technik bereits zahlreich beschriebenen Carotinoid-Formulierungen besteht weiterhin Bedarf nach Verbesserungen dieser Zubereitungen, sei es im Hinblick auf eine bessere Lagerstabilität, eine erhöhte Bioverfügbarkeit oder eine bessere Löslichkeit/Redispergierbarkeit in wässrigen Systemen, beispielsweise in Getränken.

Aufgabe der vorliegenden Erfindung war es daher, Verfahren zur Herstellung von Carotinoid-haltigen Trockenpulvern vorzuschlagen, die die oben genannten Anforderungen erfüllen.

Diese Aufgabe wurde erfindungsgemäß gelöst mit einem Verfahren zur Herstellung von Trockenpulvern eines oder mehrerer Carotinoide, dadurch gekennzeichnet, dass man
a) ein oder mehrere Carotinoide in eine wässrige molekulardisperse oder kolloiddisperse Lösung eines Gemisches aus Isomalt und mindestens einem Schutzkolloid dispergiert und
b) die gebildete Dispersion durch Abtrennung des Wassers und gegebenenfalls zusätzlich verwendeter Lösungsmittel und anschließende Trocknung, gegebenenfalls in Gegenwart eines Überzugsmaterials, in ein Trockenpulver überführt.

Als Carotinoide kommen im Rahmen der vorliegenden Erfindung u.a. α- und β-Carotin, Lycopin, Lutein, Astaxanthin, Zeaxanthin, Capsanthin, Capsorubin, α- und β-Cryptoxanthin, Citranaxanthin, Canthaxanthin, Bixin, β-Apo-4-carotinal, β-Apo-8-carotinal und β-Apo-8-carotinsäureester oder Mischungen davon in Frage. Bevorzugte Carotinoide sind β-Carotin, β-Cryptoxanthin, Lycopin, Lutein, Astaxanthin, Zeaxanthin und Canthaxanthin. Besonders bevorzugt sind Carotinoide, ausgewählt aus der Gruppe, bestehend aus β-Carotin, Lutein, Zeaxanthin und Lycopin oder Mischungen davon, ganz besonders bevorzugt sind β-Carotin, Lycopin und Lutein oder Mischungen davon, insbesondere β-Carotin.

Die Bezeichnung Isomalt steht für einen Zuckeraustauschstoff, der auch unter dem Markennamen Palatinit^{®} (Fa. Südzucker, Deutschland) geführt wird. Isomalt ist eine hydrierte Isomaltulose, die aus etwa gleichen Teilen 6-O-α-D-Glucopyranosyl-D-sorbit und 1-O-α-D-Glucopyranosyl-D-mannit besteht.

Unter einer Dispersion sind im Rahmen der vorliegenden Erfindung sowohl Emulsionen als auch Suspension, bevorzugt Suspensionen gemeint.

Als Schutzkolloide kommen beispielsweise die folgenden Stoffe in Frage:

Rinder-, Schweine- oder Fischgelatine, insbesondere sauer oder basisch abgebaute Gelatine mit Bloom-Zahlen im Bereich von 0 bis 250, ganz besonders bevorzugt Gelatine A 100, A 200, A 240, B 100 und B 200 sowie niedermolekulare, enzymatisch abgebaute Gelatinetypen mit der Bloom-Zahl 0 und Molekulargewichten von 15.000 bis 25.000 D wie zum Beispiel Collagel A und Gelitasol P (Firma Stoess, Eberbach) sowie Mischungen dieser Gelatine-Sorten.

Stärke, modifizierte Stärke, Dextrin, Pektin, Gummiarabicum, Ligninsulfonate, Chitosan, Polystyrolsulfonat, Alginate, Casein, Caseinat, Methylcellulose, Carboxymethylcellulose, Hydroxypropylcellulose oder Mischungen dieser Schutzkolloide.

Pflanzenproteine wie Soja-, Reis- und/oder Weizenproteine, wobei diese Pflanzenproteine teilabgebaut oder in nicht abgebauter Form vorliegen können.

Als bevorzugte Schutzkolloide werden im Rahmen der vorliegenden Erfindung modifizierte Stärke, insbesondere Octenylsuccinat-Stärke verwendet.

Eine bevorzugte Ausführungsform des o.g. Verfahrens ist dadurch gekennzeichnet, dass man eine im Verfahrensschritt a) hergestellte Suspension vor der Überführung in ein Trockenpulver mahlt. In diesem Fall wird der Wirkstoff [das/die Carotinoid(e)] vor dem Mahlvorgang bevorzugt in kristalliner Form in die oben genannte Schutzkolloidlösung suspendiert.

Die Mahlung kann dabei in an sich bekannter Weise z.B. mit einer Kugelmühle erfolgen. Dabei wird je nach verwendetem Mühlentyp so lange gemahlen, bis die Teilchen eine über Fraunhofer Beugung ermittelte mittlere Partikelgröße D[4,3] von 0,02 bis 100 µm, bevorzugt 0,05 bis 50 µm, besonders bevorzugt 0,05 bis 20 µm, ganz besonders bevorzugt 0,05 bis 5 µm, insbesondere 0,05 bis 0,8 µm aufweisen. Der Begriff D[4,3] bezeichnet den volumengewichteten mittleren Durchmesser (siehe Handbuch zu Malvem Mastersizer S, Malvern Instruments Ltd., UK).

Nähere Einzelheiten zur Mahlung und den dafür verwendeten Apparaturen finden sich u.a. in Ullmann's Encyclopedia of Industrial Chemistry, Sixth Edition, 2000, Electronic Release, Size Reduction, Kapitel 3.6.: Wet Grinding sowie in EP-A-0 498 824.

Die Mahlung der Carotinoidkristalle in der wässrigen Schutzkolloidlösung kann dabei sowohl in Gegenwart als auch in Abwesenheit von Isomalt erfolgen.

Eine weitere bevorzugte Ausführungsform der Erfindung ist daher auch ein Verfahren zur Herstellung eines Trockenpulvers, enthaltend Carotinoide, ausgewählt aus der Gruppe, bestehend aus β-Carotin, Lutein, Zeaxanthin und Lycopin oder Mischungen davon, dadurch gekennzeichnet, dass man
a) β-Carotin, Lutein, Zeaxanthin oder Lycopin oder Mischungen davon in eine wässrige molekulardisperse oder kolloiddisperse Lösung eines Gemisches von Isomalt mit modifizierter Stärke suspensiert,
b) die suspendierten Teilchen mahlt und
c) die Suspension anschließend in ein Trockenpulver überführt.

Ein weiterer bevorzugter Gegenstand der Erfindung ist ebenfalls ein Verfahren zur Herstellung eines Trockenpulvers, enthaltend Carotinoide, ausgewählt aus der Gruppe, bestehend aus β-Carotin, Lutein, Zeaxanthin und Lycopin oder Mischungen davon, dadurch gekennzeichnet, dass man
a) β-Carotin, Lutein, Zeaxanthin oder Lycopin oder Mischungen davon in eine wässrige molekulardisperse oder kolloiddisperse Lösung von modifizierter Stärke suspensiert,
b₁) die suspendierten Teilchen mahlt,
b₂) die gemahlene Suspension mit Isomalt mischt und
c) die Suspension anschließend in ein Trockenpulver überführt.

Eine ebenfalls bevorzugte Variante des erfindungsgemäßen Verfahrens ist dadurch gekennzeichnet, dass das Suspendieren in der Stufe a) folgende Schritte enthält:
a₁) Lösen eines oder mehrerer Carotinoide in einem mit Wasser mischbaren, organischen Lösungsmittel oder in einer Mischung aus Wasser und einem mit Wasser mischbaren, organischen Lösungsmittel oder
a₂) Lösen eines oder mehrerer Carotinoide in einem mit Wasser nicht mischbaren, organischen Lösungsmittel und
a₃) Mischen der nach a₁) oder a₂) erhaltenen Lösung mit einer wässrigen molekulardispersen oder kolloiddispersen Lösung eines Gemisches aus Isomalt und mindestens einem Schutzkolloid, wobei die hydrophobe Phase des Carotinoids als nanodisperse Phase entsteht.

Die in der Stufe a₁) verwendeten wassermischbaren Lösungsmittel sind vor allem wassermischbare, thermisch stabile, flüchtige, nur Kohlenstoff, Wasserstoff und Sauerstoff enthaltenene Lösungsmittel wie Alkohole, Ether, Ester, Ketone und Acetale zu nennen. Zweckmäßig verwendet man solche Lösungsmittel, die mindestens zu 10% wassermischbar sind, einen Siedepunkt unter 200°C aufweisen und/oder weniger als 10 Kohlenstoffe haben. Besonders bevorzugt werden Methanol, Ethanol, n-Propanol, Isopropanol, 1,2-Butandiol-1-methylether, 1,2-Propandiol-1-n-propyfether, Tetrahydrofuran oder Aceton verwendet.

Der Begriff "ein mit Wasser nicht mischbares organisches Lösungsmittel" steht im Sinne der vorliegenden Erfindung für ein organisches Lösungsmittel mit einer Wasserlöslichkeit bei Normaldruck von weniger als 10 %. Als mögliche Lösungsmittel kommen dabei u.a. halogenierte aliphatische Kohlenwasserstoffe, wie z.B. Methylenchlorid, Chloroform und Tetrachlorkohlenstoff, Carbonsäureester wie Dimethylcarbonat, Diethylcarbonat, Propylencarbonat, Ethylformiat, Methyl-, Ethyl- oder Isopropylacetat sowie Ether wie Methyl-tert. butylether in Frage. Bevorzugte, mit Wasser nicht mischbare organische Lösungsmittel sind die folgenden Verbindungen aus der Gruppe, bestehend aus Dimethylcarbonat, Propylencarbonat, Ethylformiat, Ethylacetat, Isopropylacetat und Methyl-tert. butylether.

Bei dem erfindungsgemäßen Verfahren handelt es sich bevorzugt um die Herstellung von Trockenpulvern eines oder mehrerer Carotinoide, ausgewählt aus der Gruppe, bestehend aus β-Carotin, Lutein, Zeaxanthin und Lycopin oder Mischungen davon.

Die Herstellung der o.g. Trockenpulver erfolgt vorteilhafterweise so, dass man mindestens eines der Carotinoide in einem mit Wasser mischbaren, organischen Lösungsmittel bei Temperaturen größer 30°C, vorzugsweise zwischen 50°C und 240°C, insbesondere 100°C bis 200°C, besonders bevorzugt 140°C bis 180°C, gegebenenfalls unter Druck, löst.

Da die Einwirkung hoher Temperaturen unter Umständen den gewünschten hohen alltrans Isomerenanteil herabsetzen kann, löst man das/die Carotinoid(e) möglichst rasch, beispielsweise im Sekundenbereich, z.B. in 0,1 bis 10 Sekunden, besonders bevorzugt in weniger als 1 Sekunde. Zur raschen Herstellung der molekulardispersen Lösung kann die Anwendung von erhöhtem Druck, z.B. im Bereich von 20 bar bis 80 bar, vorzugsweise 30 bis 60 bar, vorteilhaft sein.

Die so erhaltene molekulardisperse Lösung versetzt man anschließend direkt mit der gegebenenfalls gekühlten wässrigen molekulardispersen oder kolloiddispersen Lösung des Gemisches aus Isomalt und mindestens einem Schutzkolloid in der Weise, dass sich eine Mischungstemperatur von etwa 35°C bis 80°C einstellt.

Dabei wird die Lösungsmittelkomponente in die wässrige Phase überführt und die hydrophobe Phase des/der Carotinoid(e) entsteht als nanodisperse Phase.

Hinsichtlich einer näheren Verfahrens- und Apparatebeschreibung zur oben genannten Dispergierung wird an dieser Stelle auf EP-B-0 065 193 Bezug genommen.

Gegenstand der Erfindung ist ebenfalls ein Verfahren zur Herstellung eines Trockenpulvers, enthaltend Carotinoide, ausgewählt aus der Gruppe, bestehend aus β-Carotin, Lutein, Zeaxanthin und Lycopin oder Mischungen davon, dadurch gekennzeichnet, dass man
a) β-Carotin, Lutein, Zeaxanthin oder Lycopin oder Mischungen davon in einem mit Wasser mischbaren, organischen Lösungsmittel oder einer Mischung aus Wasser und einem mit Wasser mischbaren, organischen Lösungsmittel bei Temperaturen größer 30°C löst,
b) die erhaltene Lösung mit einer wässrigen molekulardispersen oder kolloiddispersen Lösung eines Gemisches von Isomalt mit modifizierter Stärke, insbesondere mit Octenylsuccinat-Stärke mischt und
c) die gebildete Suspension in ein Trockenpulver überführt.

Ganz besonders bevorzugt handelt es sich hierbei um ein Verfahren zur Herstellung β-Carotin-haltiger Trockenpulver unter Verwendung eines Gemisches aus Isomalt und modifizierte Stärke, insbesondere aus Isomalt und Octenylsuccinat-Stärke.

Die Überführung in ein Trockenpulver kann dabei u.a. durch Sprühtrocknung, Sprühkühlung, modifizierte Sprühtrocknung, Gefriertrocknung oder Trocknung im Wirbelbett, gegebenenfalls auch in Gegenwart eines Überzugsmaterials erfolgen. Als Überzugsmittel eignen sich u.a. Maisstärke, Kieselsäure oder auch Tricalciumphosphat.

Nähere Einzelheiten zur Sprühkühlung und zur modifizierten Sprühtrocknung finden sich in WO 91/06292 (Seiten 5 bis 8).

Zur Erhöhung der Stabilität des Wirkstoffes ist es vorteilhaft, Stabilisatoren wie α-Tocopherol, t-Butyl-hydroxy-toluol, t-Butylhydroxyanisol, Zitronensäure, Natriumcitrat, Ascorbinsäure, Natriumascorbat, Ascorbylpalmitat oder Ethoxyquin oder Mischungen davon in einer Konzentration von 0,05 bis 10 Gew.-%, bevorzugt 0,1 bis 7 Gew.-%, bezogen auf die Trockenmasse des Pulvers, zuzusetzen. Sie können entweder der wässrigen oder der Lösungsmittel-Phase zugesetzt werden.

Zur Erhöhung der Stabilität des Wirkstoffes gegen mikrobiellem Abbau kann es zweckmäßig sein, der Zubereitung Konservierungsmittel wie z.B. Methyl-4-hydroxybenzoat, Propyl-4-hydroxybenzoat, Sorbinsäure oder Benzoesäure oder deren Salze zuzusetzen.

Unter Umständen kann es auch vorteilhaft sein, zusätzlich in der Lösungsmittel-Phase ein physiologisch zugelassenes Öl wie beispielsweise Sesamöl, Maiskeimöl, Baumwollsaatöl, Sojabohnenöl oder Erdnußöl sowie Ester mittelkettiger pflanzlicher Fettsäuren in einer Konzentration von 0 bis 500 Gew.%, vorzugsweise 10 bis 300 Gew.%, besonders bevorzugt 20 bis 100 Gew.%, bezogen auf das/die Carotinoid(e), zu lösen, das dann gemeinsam mit den Wirkstoffen und den genannten Zusatzstoffen beim Mischen mit der wässrigen Phase extrem feinteilig ausgefällt wird.

Das Verhältnis Schutzkolloid und Isomalt zu Carotinoid wird im allgemeinen so gewählt, dass ein Endprodukt erhalten wird, das 0,1 bis 40 Gew.%, bevorzugt 1 bis 35 Gew.-%, besonders bevorzugt 5 bis 25 Gew.-% mindestens eines Carotinoids, 1 bis 50 Gew.%, bevorzugt 5 bis 40 Gew.-%, besonders bevorzugt 10 bis 35 Gew.-% mindestens eines Schutzkolloids und 10 bis 80 Gew.%, bevorzugt 15 bis 75 Gew.%, besonders bevorzugt 20 bis 60 Ges.-% Isomalt, alle Prozentangaben bezogen auf die Trockenmasse des Pulvers, sowie gegebenenfalls geringe Mengen Stabilisatoren und Konservierungsstoffe enthält.

Die Erfindung betrifft auch Trockenpulver von Carotinoiden, erhältlich nach einem der eingangs genannten Verfahren.

Bevorzugt handelt es sich dabei um Trockenpulver, enthaltend Carotinoide, ausgewählt aus der Gruppe, bestehend aus Astaxanthin, Canthaxanthin, β-Carotin, Lutein, Zeaxanthin, β-Cryptoxanthin und Lycopin, besonders bevorzugt um Trockenpulver, enthaltend ein Gemisch aus β-Carotin, Lutein, Zeaxanthin und Lycopin, ganz besonders bevorzugt um ein β-Carotin, Lutein und Lycopin-haltiges Trockenpulver, insbesondere um ein β-Carotin Trockenpulver.

Die erfindungsgemäßen Trockenpulver zeichnen sich u.a. dadurch aus, dass sie sich in wässrigen Systemen unter Erzielung einer gleichmäßigen Feinverteilung des Wirkstoffes im Korngrößenbereich kleiner 1 µm problemlos wieder redispergieren lassen.

Die Verwendung einer Kombination aus Isomalt und Schutzkolloiden, insbesondere modifizierte Stärke als Formutierhilfsstoffe hat gegenüber anderen Zuckern, beispielsweise Lactose oder Sucrose den Vorteil, dass die damit hergestellten Carotinoid-Formulierungen eine besonders hohe Lagerstabilität u.a. in Multivitamintabletten zeigen (siehe Tabelle).

Die erfindungsgemäßen Carotinoid-Formulierungen eignen sich u.a. als Zusatzstoff zu Lebensmittelzubereitungen, beispielsweise zur Färbung von Lebensmitteln wie Getränken, als Mittel für die Herstellung pharmazeutischer und kosmetischer Zubereitungen sowie für die Herstellung von Nahrungsergänzungspräparaten, beispielsweise von Multivitaminpräparaten im Human- und Tierbereich.

In den nachfolgenden Beispielen wird die Durchführung des erfindungsgemäßen Verfahrens näher erläutert.

### Beispiel 1

### Herstellung eines β-Carotin Trockenpulvers unter Verwendung einer Mischung aus Isomalt und Octenylsuccinatstärke

a.
   Unter Schutzgas wurden 19,5 I Wasser auf 55°C erwärmt und mit 0,44 kg Natriumascorbat, 0,39 kg Ascorbinsäure und 8,33 kg Octenylsuccinatstärke (Capsul^{®}, Fa. National Starch) versetzt. In diese Lösung wurden unter Rühren 8,33 kg kristallines β-Carotin einsuspendiert. Anschließend wurde die Suspension mit Hilfe einer Kugelmühle so lange gemahlen, bis die β-Carotinteilchen eine über Fraunhofer Beugung ermittelte mittlere Partikelgröße D[4,3] von kleiner 0,8 µm aufwiesen.
b.
   2,93 kg dieser gemahlenen Suspension wurden unter Schutzgas in einen zweiten Reaktor überführt und unter Rühren mit 0,75 kg Isomalt und weiteren 0,456 kg Octenylsuccinatstärke versetzt. Die Temperatur dieser Mischung wurde auf 55°C gehalten. Nach Zugabe von 0,0335 kg α-Tocopherol wurde die Suspension homogenisiert und anschließend durch modifizierte Sprühtrocknung in ein Trockenpulver in Form von Beadlets überführt. Der Gehalt an β-Carotin in den Beadlets betrug 21,0 % bei einem E1/1-Wert¹⁾ von 85.

### Beispiel 2

### Herstellung eines β-Carotin Trockenpulvers unter Verwendung einer Mischung aus Isomalt und Octenylsuccinatstärke

Unter Schutzgas werden 1,97 I Wasser auf 55°C erwärmt und mit 26,5 g Natriumascorbat, 23,5 g Ascorbinsäure, 564 g Isomalt und 500 g Octenylsuccinatstärke (Capsul^{®}, Fa. National Starch) versetzt. In diese Lösung werden unter Rühren 500 g kristallines β-Carotin einsuspendiert und die Suspension wird mit Hilfe einer Kugelmühle so lange gemahlen, bis die β-Carotinteilchen eine über Fraunhofer Beugung ermittelte mittlere Partikelgröße D[4,3] von kleiner 0,8 µm aufweisen.

Anschließend werden weitere 340,7 g Octenylsuccinatstärke in der Suspension gelöst. Nach Zugabe von 25 g α-Tocopherol wird die Suspension homogenisiert und durch modifizierte Sprühtrocknung in ein Trockenpulver in Form von Beadlets überführt. Der Gehalt an β-Carotin in den Beadlets beträgt 20 %.

### Beispiel 3

### Herstellung eines Trockenpulvers enthaltend β-Carotin, Lutein und Lycopin unter Verwendung einer Mischung aus Isomalt und Octenylsuccinatstärke

Unter Schutzgas werden 2,4 I Wasser auf 55°C erwärmt und mit 26,5 g Natriumascorbat, 23,5 g Ascorbinsäure, 564 g Isomalt und 500 g Octenylsuccinatstärke (Capsul^{®}, Fa. National Starch) versetzt. In diese Lösung werden unter Rühren 167 g kristallines β-Carotin, 167 g kristallines Lycopin und 167 g kristallines Lutein einsuspendiert und die Suspension mit Hilfe einer Kugelmühle so lange gemahlen, bis die Carotinoidteilchen eine über Fraunhofer Beugung ermittelte mittlere Partikelgröße D[4,3] von kleiner 0,8 µm aufweisen.

Weitere 1420 g Octenylsuccinatstärke und 715 g Isomalt werden in der gemahlenen Suspension gelöst. Nach Zugabe von 25 g α-Tocopherol wird die Suspension homogenisiert und anschließend durch modifizierte Sprühtrocknung in ein Trockenpulver in Form von Beadlets überführt. Der Gesamtgehalt an Carotinoiden in den Beadlets beträgt 10 % mit einem Verhältnis β-Carotin : Lutein : Lycopin von 1:1:1.

### Beispiel 4 (Vergleichsversuch)

### Herstellung eines β-Carotin Trockenpulvers unter Verwendung einer Mischung aus Trehalose und Octenylsuccinatstärke

3,11 kg der gemahlenen Suspension aus Beispiel 1a. wurden unter Schutzgas in einen zweiten Reaktor überführt und unter Rühren mit 0,8 kg Trehalose und weiteren 0,456 kg Octenylsuccinatstärke versetzt. Die Temperatur dieser Mischung wurde auf 55°C gehalten. Nach Zugabe von 0,036 kg α-Tocopherol wurde die Suspension homogenisiert und anschließend durch modifizierte Sprühtrocknung in ein Trockenpulver in Form von Beadlets überführt. Der Gehalt an β-Carotin in den Beadlets betrug 23,6 % bei einem E1/1-Wert¹⁾ von 84.

### Beispiel 5 (Vergleichsversuch)

### Herstellung eines β-Carotin Trockenpulvers unter Verwendung einer Mischung aus Mannitol und Octenylsuccinatstärke

3,32 kg der gemahlenen Suspension aus Beispiel 1a. wurden unter Schutzgas in einen zweiten Reaktor überführt und unter Rühren mit 0,85 kg Mannitol und weiteren 0,517 kg Octenylsuccinatstärke versetzt. Die Temperatur dieser Mischung wurde auf 55°C gehalten. Nach Zugabe von 0,038 kg α-Tocopherol wurde die Suspension homogenisiert und anschließend durch modifizierte Sprühtrocknung in ein Trockenpulver in Form von Beadlets überführt. Der Gehalt an β-Carotin in den Beadlets betrug 21,7 % bei einem E1/1-Wert¹⁾ von 78.

### Beispiel 6 (Vergleichsversuch)

### Herstellung eines β-Carotin Trockenpulvers unter Verwendung einer Mischung aus Sucrose und Octenylsuccinatstärke

2,56 kg der gemahlenen Suspension aus Beispiel 1 a. wurden unter Schutzgas in einen zweiten Reaktor überführt und unter Rühren mit 0,66 kg Saccharose und weiteren 0,4 kg Octenylsuccinatstärke versetzt. Die Temperatur dieser Mischung wurde auf 55°C gehalten. Nach Zugabe von 0,030 kg α-Tocopherol wurde die Suspension homogenisiert und anschließend durch modifizierte Sprühtrocknung in ein Trockenpulver in Form von Beadlets überführt. Der Gehalt an β-Carotin in den Beadlets betrug 21,6 % bei einem E1/1-Wert¹⁾ von 89.

### Beispiel 7 (Vergleichsversuch)

### Herstellung eines β-Carotin Trockenpulvers unter Verwendung von Octenylsuccinatstärke

2,82 kg der gemahlenen Suspension aus Beispiel 1 a. wurden unter Schutzgas in einen zweiten Reaktor überführt und unter Rühren mit weiteren 1,15 kg Octenylsuccinatstärke versetzt. Die Temperatur dieser Mischung wurde auf 55°C gehalten. Nach Zugabe von 0,030 kg α-Tocopherol wurde die Suspension homogenisiert und anschließend durch modifizierte Sprühtrocknung in ein Trockenpulver in Form von Beadlets überführt. Der Gehalt an β-Carotin in den Beadlets betrug 23,1 % bei einem E1/1-Wert¹⁾ von 90.

### Beispiel 8

### Herstellung eines β-Carotin Trockenpulvers unter Verwendung einer Mischung aus Isomalt und Natriumcaseinat

In einer beheizbaren Vorlage wurden 62 g kristallines β-Carotin, 20 g α-Tocopherol und 5 g Ascorbylpalmitat in 430 g eines azeotropen Isopropanol/Wasser-Gemischs bei Raumtemperatur suspendiert. Die Wirkstoffsuspension wurde dann auf 90°C erwärmt und bei einer Flussrate von 2,9 kg/h kontinuierlich mit weiterem Isopropanol/Wasser-Azeotrop der Temperatur 220°C und einer Flussrate von 4,5 kg/h vermischt, wobei sich β-Carotin bei einer sich einstellenden Mischungstemperatur von 175°C bei einem Druck von 55 bar auflöste. Diese Wirkstofflösung wurde unmittelbar anschließend mit einer wässrigen Phase, bestehend aus einer Lösung von 75 g Natriumcaseinat, 290,5 g Isomalt und 10 g Konservierungsmittel in 8325 g destilliertem Wasser, in dem der pH-Wert mit 1 M NaOH auf pH 9,5 eingestellt wurde, bei einer Flussrate von 50 kg/h vermischt.

Die bei der Mischung entstandenen Wirkstoffteilchen wiesen im Isopropanoi/Wasser-Gemisch eine Teilchengröße von 180 nm auf, bei einem E1/1-Wert¹⁾ von 117.

Anschließend wurde die Wirkstoff-Suspension am Dünnfilmverdampfer auf eine Konzentration von ca. 30 Gew.-% Trockengehalt aufkonzentriert und sprühgetrocknet. Das Trockenpulver wies einen β-Carotin-Gehalt von 13,2 Gew.-% auf. Das in Wasser redispergierte Trockenpulver hatte eine Teilchengröße von 190 nm und wies einen E1/1-Wert von 116 auf.

### Beispiel 9

### Herstellung eines β-Carotin Trockenpulvers unter Verwendung einer Mischung aus Isomalt und Octenylsuccinatstärke

In einer beheizbaren Vorlage wurden 62 g kristallines β-Carotin und 19 g α-Tocopherol in 430 g eines azeotropen Isopropanol/Wasser-Gemischs bei Raumtemperatur suspendiert. Die Wirkstoffsuspension wurde dann auf 90°C erwärmt und bei einer Flussrate von 2,9 kg/h kontinuierlich mit weiterem Isopropanol/Wasser-Azeotrop der Temperatur 220°C und einer Flussrate von 4,5 kg/h vermischt, wobei sich β-Carotin bei einer sich einstellenden Mischungstemperatur von 175°C bei einem Druck von 55 bar auflöste. Diese Wirkstofflösung wurde unmittelbar anschließend mit einer wässrigen Phase, bestehend aus einer Lösung von 160 g Capsul, 220,5 g Isomalt und 10 g Konservierungsmittel in 8325 g destilliertem Wasser, bei einer Flussrate von 50 kg/h vermischt.

Die bei der Mischung entstandenen Wirkstoffteilchen wiesen im Isopropanol/Wasser-Gemisch eine Teilchengröße von 250 nm auf, bei einem E1/1-Wert¹⁾ von 95.
¹⁾ Der E1/1-Wert definiert in diesem Zusammenhang die spezifische Extinktion einer 0,5%igen wässrigen Dispersion eines 20 Gew.-%igen Trockenpulvers in einer 1 cm-Küvette im Absorptionsmaximum.

Anschließend wurde die Wirkstoff-Suspension am Dünnfilmverdampfer auf eine Konzentration von ca. 35 Gew.-% Trockengehalt aufkonzentriert und sprühgetrocknet. Das Trockenpulver wies einen β-Carotin-Gehalt von 13,0 Gew.-% auf. Das in Wasser redispergierte Trockenpulver hatte eine Teilchengröße von 252 nm und wies einen E1/1-Wert von 93 auf.

### Tabelle: Lagerstabilität der β-Carotin Beadlets in Multivitamin Tabletten

Die Stabilität der β-Carotin Beadlets wurde getestet anhand von Multivitamin-Mineral-Tabletten mit einem Gehalt von ca. 3 mg β-Carotin pro Tablette. Die Tabletten wurden in HDPE-Behältern verpackt, deren Deckel mit eingeschweister Aluminium Folie abgedichtet war. Die Lagerung der Tabletten erfolgte 6 Monate bei 40°C und 75% relativer Luftfeuchtigkeit. Der Gehalt an β-Carotin wurde jeweils nach 3 und 6 Monaten Lagerzeit analysiert.

**Tabelle**

| | | | | t = 0 Monate | Nach 3 Monaten | | Nach 6 Monaten | |
|---|---|---|---|---|---|---|---|---|
| Bsp. | Zucker | Kolloid | Gehalt: β-Carotin | Gehalt β-Carotin pro Tablette [mg] | Gehalt β-Carotin pro Tablette [mg] | Verlust (%) | Gehalt β-Carotin pro Tablette [mg] | Verlust (%) |
| 1 | Isomalt | Capsul | 21,0 | 3,40 | 2,77 | 18,5 | 2,68 | 21,2 |
| 4 | Trehalose | Capsul | 23,6 | 3,77 | 2,72 | 27,9 | 2,59 | 31,3 |
| 5 | Mannitol | Capsul | 21,7 | 3,46 | 2,11 | 39,0 | 2,12 | 38,7 |
| 6 | Sucrose | Capsul | 21,6 | 3,39 | 2,57 | 24,2 | 2,45 | 27,7 |
| 7 | - | Capsul | 23,1 | 3,63 | 2,42 | 33,3 | | |

## Patentansprüche

1. Verfahren zur Herstellung von Trockenpulvern eines oder mehrerer Carotinoide, **dadurch gekennzeichnet, dass** man
a) ein oder mehrere Carotinoide in eine wässrige molekulardisperse oder kolloiddisperse Lösung eines Gemisches aus Isomalt und mindestens einem Schutzkolloid dispergiert und
b) die gebildete Dispersion durch Abtrennung des Wassers und gegebenenfalls zusätzlich verwendeter Lösungsmittel und anschließende Trocknung, gegebenenfalls in Gegenwart eines Überzugsmaterials, in ein Trockenpulver überführt.

2. Verfahren nach Anspruch 1, **dadurch gekennzeichnet, dass** es sich bei der Dispersion um eine Suspension handelt.

3. Verfahren nach Anspruch 2, **dadurch gekennzeichnet, dass** man die im Verfahrensschritt a) hergestellte Suspension vor der Überführung in ein Trockenpulver mahlt.

4. Verfahren nach Anspruch 2, **dadurch gekennzeichnet, dass** das Suspendieren im Verfahrensschritt a) folgende Schritte umfaßt:
a₁) Lösen eines oder mehrerer Carotinoide in einem mit Wasser mischbaren, organischen Lösungsmittel oder in einer Mischung aus Wasser und einem mit Wasser mischbaren, organischen Lösungsmittel oder
a₂) Lösen eines oder mehrerer Carotinoide in einem mit Wasser nicht mischbaren, organischen Lösungsmittel und
a₃) Mischen der nach a₁) oder a₂) erhaltenen Lösung mit einer wässrigen molekulardispersen oder kolloiddispersen Lösung eines Gemisches aus Isomalt und mindestens einem Schutzkolloid, wobei die hydrophobe Phase des Carotinoids als nanodisperse Phase entsteht.

5. Verfahren nach einem der Ansprüche 1 bis 4, **dadurch gekennzeichnet, dass** man als Schutzkolloid modifizierte Stärke verwendet.

6. Verfahren nach einem der Ansprüche 1 bis 5, **dadurch gekennzeichnet, dass** es sich bei den verwendeten Carotinoiden um Verbindungen, ausgewählt aus der Gruppe, bestehend aus β-Carotin, Lutein, Zeaxanthin und Lycopin oder Mischungen davon handelt.

7. Verfahren zur Herstellung eines Trockenpulvers, enthaltend Carotinoide, ausgewählt aus der Gruppe, bestehend aus β-Carotin, Lutein, Zeaxanthin und Lycopin oder Mischungen davon, **dadurch gekennzeichnet, dass** man
a) β-Carotin, Lutein, Zeaxanthin oder Lycopin oder Mischungen davon in einem mit Wasser mischbaren, organischen Lösungsmittel oder einer Mischung aus Wasser und einem mit Wasser mischbaren, organischen Lösungsmittel bei Temperaturen größer 30°C löst,
b) die erhaltene Lösung mit einer wässrigen molekulardispersen oder kolloiddispersen Lösung eines Gemisches von Isomalt mit modifizierter Stärke mischt und
c) die gebildete Suspension in ein Trockenpulver überführt.

8. Verfahren zur Herstellung eines Trockenpulvers, enthaltend Carotinoide, ausgewählt aus der Gruppe, bestehend aus β-Carotin, Lutein, Zeaxanthin und Lycopin oder Mischungen davon, **dadurch gekennzeichnet, dass** man
a) β-Carotin, Lutein, Zeaxanthin oder Lycopin oder Mischungen davon in eine wässrige molekulardisperse oder kolloiddisperse Lösung eines Gemisches von Isomalt mit modifizierter Stärke suspensiert,
b) die suspendierten Teilchen mahlt und
c) die Suspension anschließend in ein Trockenpulver überführt.

9. Verfahren zur Herstellung eines Trockenpulvers, enthaltend Carotinoide, ausgewählt aus der Gruppe, bestehend aus β-Carotin, Lutein, Zeaxanthin und Lycopin oder Mischungen davon, **dadurch gekennzeichnet, dass** man
a) β-Carotin, Lutein, Zeaxanthin oder Lycopin oder Mischungen davon in eine wässrige molekulardisperse oder kolloiddisperse Lösung von modifizierter Stärke suspensiert,
b₁) die suspendierten Teilchen mahlt,
b₂) die gemahlene Suspension mit Isomalt mischt und
c) die Suspension anschließend in ein Trockenpulver überführt.

10. Verfahren nach einem der Ansprüche 7 bis 9, **dadurch gekennzeichnet, dass** man als Schutzkolloid ein Gemisch aus Isomalt und Octenylsuccinat-Stärke verwendet.

11. Carotinoid-haltige Trockenpulver, erhältlich nach einem Verfahren, definiert gemäß einem der Ansprüche 1 bis 10.

12. Trockenpulver nach Anspruch 11 mit einem Carotinoidgehalt von 0,1 bis 40 Gew.%.

13. Trockenpulver nach Anspruch 12, enthaltend 5 bis 25 Gew.-% Carotinoide, ausgewählt aus der Gruppe, bestehend aus β-Carotin, Lutein, Zeaxanthin und Lycopin oder Mischungen davon.

14. Verwendung der Carotinoid-haltigen Trockenpulver, definiert gemäß einem der Ansprüche 11 bis 13, als Zusatz zu Lebensmitteln, Pharmazeutika und/oder Tierfuttermitteln.

## Claims

1. A process for producing dry powders of one or more carotenoids, which comprises
a) dispersing one or more carotenoids in an aqueous molecular or colloidal solution of a mixture of isomalt and at least one protective colloid and
b) converting the dispersion which has formed into a dry powder by removing the water and, if appropriate, additionally used solvents and subsequently drying, if appropriate in the presence of a coating material.

2. The process according to claim 1, wherein the dispersion is a suspension.

3. The process according to claim 2, wherein the suspension produced in process step a) is ground before conversion into a dry powder.

4. The process according to claim 2, wherein the suspending in process step a) comprises the following steps:
a₁) dissolving one or more carotenoids in a water-miscible organic solvent or in a mixture of water and a water-miscible organic solvent or
a₂) dissolving one or more carotenoids in a water-immiscible organic solvent and
a₃) mixing the solution obtained as in a₁) or a₂) with an aqueous molecular or colloidal solution of a mixture of isomalt and at least one protective colloid, resulting in the hydrophobic phase of the carotenoid as nanodisperse phase.

5. The process according to any of claims 1 to 4, wherein modified starch is used as protective colloid.

6. The process according to any of claims 1 to 5, wherein the carotenoids used are compounds selected from the group consisting of β-carotene, lutein, zeaxanthin and lycopene or mixtures thereof.

7. A process for producing a dry powder comprising carotenoids selected from the group consisting of β-caratene, lutein, zeaxanthin and lycopene or mixtures thereof, which comprises
a) dissolving β-carotene, lutein, zeaxanthin or lycopene or mixtures thereof in a water-miscible organic solvent or a mixture of water and a water-miscible organic solvent at temperatures above 30°C,
b) mixing the resulting solution with an aqueous molecular or colloidal solution of a mixture of isomalt with modified starch and
c) converting the suspension which has formed into a dry powder.

8. A process for producing a dry powder comprising carotenoids selected from the group consisting of β-carotene, lutein, zeaxanthin and lycopene or mixtures thereof, which comprises
a) suspending β-carotene, lutein, zeaxanthin or lycopene or mixtures thereof in an aqueous molecular or colloidal solution of a mixture of isomalt with modified starch,
b) grinding the suspended particles and
c) converting the suspension subsequently into a dry powder.

9. A process for producing a dry powder comprising carotenoids selected from the group consisting of β-carotene, lutein, zeaxanthin and lycopene or mixtures thereof, which comprises
a) suspending β-carotene, lutein, zeaxanthin or lycopene or mixtures thereof in an aqueous molecular or colloidal solution of modified starch,
b₁) grinding the suspended particles,
b₂) mixing the ground suspension with isomalt and
c) converting the suspension subsequently into a dry powder.

10. The process according to any of claims 7 to 9, wherein a mixture of isomalt and octenylsuccinate-starch is used as protective colloid.

11. A carotenoid-containing dry powder obtainable by a process defined in any of claims 1 to 10.

12. A dry powder according to claim 11 having a carotenoid content of from 0.1 to 40% by weight.

13. The dry powder according to claim 12, comprising from 5 to 25% by weight of carotenoids selected from the group consisting of β-carotene, lutein, zeaxanthin and lycopene or mixtures thereof.

14. The use of the carotenoid-containing dry powders defined in any of claims 11 to 13 as addition to food products, pharmaceuticals and/or animal feeds.

## Revendications

1. Procédé de préparation de poudres sèches d'un ou de plusieurs caroténoïdes, **caractérisé en ce que :**
a) on disperse un ou plusieurs caroténoïdes dans la solution aqueuse de dispersion moléculaire ou de dispersion colloïdale d'un mélange d'isomalt et d'au moins un colloïde protecteur, et
b) on transforme en poudre sèche la dispersion formée par séparation de l'eau et des solvants éventuellement utilisés à titre additionnel et séchage consécutif, éventuellement en présence d'une matière d'enrobage.

2. Procédé selon la revendication 1, **caractérisé en ce qu'**il s'agit pour la dispersion, d'une suspension.

3. Procédé selon la revendication 2, **caractérisé en ce qu'**on broie la suspension issue de l'étape de procédé a) en une poudre sèche avant la transformation.

4. Procédé selon la revendication 2, **caractérisé en ce que** la mise en suspension à l'étape de procédé a) englobe les étapes suivantes :
a₁) dissolution d'un ou de plusieurs caroténoïdes dans un solvant organique miscible à l'eau ou dans un mélange d'eau et d'un solvant organique miscible à l'eau, ou
a₂) dissolution d'un ou de plusieurs caroténoïdes dans un solvant organique non miscible à l'eau, et
a₃) mélange de la solution obtenue en a₁) ou a₂) avec la solution aqueuse de dispersion moléculaire ou de dispersion colloïdale d'un mélange composté d'isomalt et d'au moins un colloïde protecteur, la phase hydrophobe du caroténoïde apparaissant sous la forme d'une phase nanodïspersée.

5. Procédé selon l'une des revendications 1 à 4, **caractérisé en ce qu'**on utilise de l'amidon codifié comme colloïde protecteur.

6. Procédé selon l'une des revendications 1 à 5, **caractérisé en ce qu'**il s'agit pour les caroténoïdes employés, de composés choisis parmi le groupe constitué de β-carotène, de lutéine, de zéaxanthine et de lycopine ou de mélanges de ceux-ci.

7. Procédé de préparation d'une poudre sèche, contenant des caroténoïdes choisis parmi le groupe constitué de β-carotène, de lutéine, de zéaxanthine et de lycopine ou de mélanges de ceux-ci, **caractérisé en ce que :**
a) on dissout le β-carotène, la lutéine, la zéaxanthine ou la lycopine ou des mélanges de ceux-ci dans un solvant organique miscible à l'eau ou dans un mélange d'eau et d'un solvant organique miscible à l'eau à des températures supérieures à 30 °C,
b) on mélange la solution obtenue avec la solution aqueuse de dispersion moléculaire ou de dispersion colloïdale d'un mélange d'isomalt avec de l'amidon modifié, et
c) on transforme la suspension formée en une poudre sèche.

8. Procédé de préparation d'une poudre sèche, contenant des caroténoïdes choisis parmi le groupe constitué de β-carotène, de lutéine, de zéaxanthine et de lycopine ou de mélanges de ceux-ci, **caractérisé en ce que :**
a) on met en suspension le β-carotène, la lutéine, la zéaxanthine ou la lycopine ou des mélanges de ceux-ci dans la solution aqueuse de dispersion moléculaire ou de dispersion colloïdale d'un mélange d'isomalt avec de l'amidon modifié,
b) on broie les particules mises en suspension, et
c) on transforme ensuite la suspension en une poudre sèche.

9. Procédé de préparation d'une poudre sèche, contenant des caroténoïdes choisis parmi le groupe constitué de β-carotène, de lutéine, de zéaxanthine et de lycopine ou de mélanges de ceux-ci, **caractérisé en ce que :**
a) on met en suspension le β-carotène, la lutéine, la zéaxanthine ou la lycopine ou des mélanges de ceux-ci dans une solution aqueuse de dispersion moléculaire ou de dispersion colloïdale d'amidon modifié,
b₁) on broie les particules mises en suspension,
b₂) on mélange la suspension broyée avec l'isomalt, et
c) on transforme ensuite la suspension en une poudre sèche.

10. Procédé selon l'une des revendications 7 à 9, **caractérisé en ce qu'**on utilise comme colloïde protecteur un mélange d'isomalt et d'octénylsuccinate-amidon.

11. Poudre sèche contenant un caroténoïde qu'on obtient selon un procédé défini selon l'une des revendications 1 à 10.

12. Poudre sèche selon la revendication 11 avec une teneur en caroténoïde de 0,1 à 40 % en poids.

13. Poudre sèche selon la revendication 12, contenant de 5 à 25 % en poids de caroténoïdes, choisis parmi le groupe constitué de β-carotène, de lutéine, de zéaxanthine et de lycopine ou des mélanges de ceux-ci.

14. Utilisation de la poudre sèche contenant un caroténoïde, définie selon l'une des revendications 11 à 13, comme additif à des produits alimentaires, à des produits pharmaceutiques et/ou à des produits d'alimentation pour animaux.
